# EUROPEAN PATENT APPLICATION

(11) **EP 4 834 097 A1**
(43) Date of publication of application: **04.11.2026**
(21) Application number: 25382425.4
(22) Date of filing: 28.04.2025
(51) Int. Cl.: A01H 1/02

(54) **POLLINATION METHOD USING ELECTROSTATIC TECHNOLOGY**

(71) Applicant: Bisari Agroinnovation, S.L., 18220 Albolote Granada (ES)
(72) Inventor: Sánchez Rodríguez, Eva, Albolote (ES)
(74) Representative: Pons IP

(57) **Abstract**

The present invention relates to a method for pollinating a plant by means of electrospraying, wherein the pollen is applied in a liquid composition comprising an osmotic component, a calcium source, and a boron source. Additionally, the composition may further comprise other elements such as amino acids, polyamines, brassinosteroids, or microorganisms.

## Description

The present invention relates to a method for pollinating plants by electrospray. Therefore, the invention could be classified within the field of agricultural biotechnology.

### STATE OF THE ART

A significant portion of global agricultural crops depends on pollination to achieve successful yields.

Natural pollination can occur through abiotic means-such as wind (anemophilous pollination), water (hydrophilous pollination), or gravity-or more commonly through biotic vectors, namely animals that transfer pollen from one flower to another, primarily specific insect species.

Artificial or assisted pollination involves human intervention in the pollination process, thereby enabling control over plant reproduction (Broussard, M. A., Coates, M., & Martinsen, P. (2023). Artificial pollination technologies: A review. Agronomy, 13(5), 1351). This practice may be necessary due to various factors, such as the scarcity of natural pollinators in a crop, reduced pollinator activity due to adverse weather conditions, the absence of viable or compatible pollen when female floral organs are receptive, or to avoid undesired changes in specific plant traits and to ensure yield and/or product quality.

Typically, the process involves bagging flowers to prevent exposure to external pollinators, collecting specific pollen from the stamen, and transferring it to the stigma, covering back the flowers until fertilization occurs.

In addition to direct contact transfer of pollen from flower to flower by means of a brush or a similar tool, there are essentially two primary methods for pollen application. First, dry pollen application also known as dry spraying, which entails a large expenditure of pollen and a high risk of drift losses, thus resulting inefficient and economically extremely costly.

Second, there is the use of liquid media through spraying or misting systems that typically employ distilled water as carrier. This allows for reduced pollen use and a more homogeneous application. However, this method compromises pollen viability due to osmotic imbalances between the external solution and the intracellular environment of the pollen grain, leading to fluid influx and potential cellular rupture, potentially causing death and limiting fertilization capability.

Furthermore, these pollination techniques often rely on conventional spraying systems adapted from other sectors, which are not optimized for pollen application. They fail to ensure efficient adherence and distribution of pollen on floral structures, thus requiring high dosages to achieve reproductive goals. This is especially problematic given the fact that pollen is a scarce and difficult-to-obtain resource, making efficient application crucial to maximizing limited supplies.

Additionally, beyond the application phase, the pollen collection and preservation processes are also critical components of the overall pollination system. Currently, there are no standardized commercial systems for pollen collection and storage. In particular, existing collection methods hinder the separation of pollen from floral debris, resulting in impurities that interfere with effective pollination.

Therefore, there is an unmet need for assisted pollination systems that enable optimized pollen concentration, use lower pollen quantities than conventional methods, incorporate media that preserve or enhance pollen viability, and ensure more effective pollen delivery to flowers. Moreover, such systems should include standardized collection and preservation modules tailored to different crops, facilitating optimized extraction for each specific case.

### DESCRIPTION OF THE INVENTION

The present invention refers to an assisted pollination method aimed to complement natural pollination in those plantations where pollination is not optimal.

The method of the present invention presents two distinctive features: i) the suspension of pollen in a liquid medium (matrix) which contains the necessary components to preserve pollen and improve its application, ii) where the application of the resulting composition by means of a spraying system with electrostatic technology.
i) The suspension of pollen in a liquid medium offers the following advantages:
   - Acting as a carrier that enables pollen transportation with a dual benefit: its inclusion in water droplets reduces drift risk, and the homogeneous diffusion throughout the liquid allows for higher application volumes while requiring less pollen. This constitutes an improvement of great importance when the limited pollen availability and the difficulty in obtaining pollen in most crop systems are taken into account.
   - Ensuring pollen integrity, preventing viability losses due to osmotic shocks. This is achieved by balancing the osmotic potential between the external medium and the interior of the pollen grain, incorporating to the matrix a specific concentration of dissolved salts that closely mimics the internal osmotic conditions of the pollen.
   - Promoting pollen fecundation capacity by germination and pollen tube elongation stimulation. To achieve this, the matrix includes three types of components which, specifically selected for the pollen of each particular crop species, have shown to reduce germination time and act as inductors of the pollen tube. Thus, grain germination is ensured and the required time for fecundation is reduced, which is of great interest given the short lifespan of the ovule.
ii) The application of the pollen is carried out using a nebulizations system incorporating electrostatic technology (electrospraying), which confers the following advantages:
   - Optimal droplet size and uniformity to reach the electrostatic effect.
   - Increased application efficiency: droplets carry an electric charge that induces an attractive force toward the plant's target surface. This results in a reduction of spray losses through atmospheric drift and ground runoff, enhancing deposition efficiency.
   - Improved uniformity distribution: the droplets' electric charge, due to sharing the same sign, generates repulsive forces among the droplets, which prevents their coalescence into larger droplets thus enabling a more homogeneous coverage of the pulverised surface. Additionally, this repulsion, combined with the system's natural tendency to neutralize global charge, produces a wrapping effect by which the droplets are distributed along all the target surface, including non-exposed areas. Moreover, the electric charge reaches a higher magnitude in protruding or angular points, such as the floral stigma, therefore droplets tend to be deposited profusely in those points.

This methodology provides a means to improve the yield of cultivated plants. Moreover, it enhances the autonomy of the pollination process by reducing its dependency on environmental conditions or the availability of natural pollen sources.

Thus, one aspect of the present invention relates to a method for pollinating a plant, hereinafter referred to as the "method of the invention", which comprises the following steps:
a) Mixing pollen with a liquid composition; and
b) Applying the mixture obtained in step a) to the plant via electrospraying; wherein the liquid composition from step a) comprises an osmotic component, a calcium source, and/or a boron source.

Pollination is the biological process by which pollen is transported from the stamens or male floral organs of a flower, where it is produced, to the stigma or female floral organs of the same or a different flower. This leads to subsequent pollen germination, pollen tube growth, and ovule fertilization, thereby enabling seed and fruit formation.

In the context of the present invention, the term *"pollen"* refers to the ensemble of pollen grains produced by seed plants (spermatophytes), each containing a male gametophyte.

Preferably, the pollen included in the liquid composition originates from a plant of the same species as the plant to be pollinated, or from a closely related species, thus enabling cross-pollination.

Pollen can be obtained through any method known to a person skilled in the art, including but not limited to aspiration, liquid-phase extraction, centrifugation, and/or floral sieving.

More preferably, pollen collection can be carried out:
- Aspiration system and hydrocyclonic separation for crops with abundant and accessible pollen, such as sunflower or pistachio.
- Two-Phase Liquid Extraction for tropical crops with small flowers, such as avocado, mango, or lychee, which typically yield scarce and highly adherent pollen:
   ∘ A first phase using the organic solvent cyclohexane to extract pollen directly from the flower.
   ∘ A second phase consisting of a 10% dilution of a crop-specific matrix in distilled water, where the pollen collected in the first phase is transferred for preservation.
- Mechanical flower collection by tree vibration, followed by a drying process which time and temperature is to be adjusted according to the degree of initial moisture content of the flowers, and a subsequent centrifugal rotation and sieving for rapid pollen separation. This method is suitable for pit and seed fruit trees.

After collection, the pollen may be analysed to detect contamination by pathogens, assess viability, and/or determine germination rate, in order to select the most suitable pollen prior to its mixing with the composition in step (a) of the method of the invention.

When immediate use is not intended, pollen may be stored, for example via cryopreservation.

The liquid composition used in step (a) of the method comprises components that increase pollen viability by preventing osmotic stress and ultimately enhancing pollination efficiency.

Specifically, the liquid composition in step (a) comprises an osmotic component, a calcium source, and a boron source.

In a preferred embodiment of the invention, the liquid composition comprises between 12% and 28% (w/v) of an osmotic component; between 0.005% and 0.10% (w/v) of a calcium source; and/or between 0.003% and 0.10% (w/v) of a boron source.

In the present invention, the term "osmotic component" refers to a compound that acts as an osmotic protectant.

In the present invention, the term "calcium source" refers to any compound containing calcium.

In the present invention, the term "boron source" refers to any compound containing boron. In a preferred embodiment, the osmotic component is a carbohydrate, the calcium source is an inorganic salt, and/or the boron source is boric acid.

Thus, in a more preferred embodiment, the liquid composition comprises between 12% and 28% (w/v) of carbohydrate; between 0.005% and 0.10% (w/v) of calcium inorganic salt; and/or between 0.003% and 0.10% (w/v) of boric acid; where all percentages are expressed as weight per volume (w/v) of the total liquid composition.

In the present invention, the term "carbohydrate" refers to organic compounds composed primarily of carbon, hydrogen, and oxygen. These include monosaccharides, disaccharides, oligosaccharides, and polysaccharides.

In a preferred embodiment of the method of the invention, the osmotic component of the liquid composition is a carbohydrate selected from a monosaccharide, a disaccharide, or a combination thereof. Examples of monosaccharides and disaccharides include, without limitation, glucose, fructose, or sucrose.

In another preferred embodiment of the method of the invention, the carbohydrate from the liquid composition is selected from the list consisting of sucrose, fructose, glucose, or a combination thereof, preferably where the carbohydrate is sucrose.

The term "inorganic salt" refers to a compound which results from the reaction of an acid and a base, where the resulting compound comprises cations and anions, and as a whole is electrically neutral. An inorganic salt lacks carbon-hydrogen (C-H) bonds typical of organic compounds. Examples of inorganic salts include, but are not limited to, nitrates, sulphates, calcium nitrate (Ca(NO₃)₂), potassium nitrate (KNO₃), and magnesium sulphate (MgSO₄).

In the present invention, a calcium inorganic salt corresponds with a salt comprising calcium in its structure.

In a preferred embodiment of the method of the invention, the calcium inorganic salt is calcium nitrate.

In another preferred embodiment of the method of the invention, the liquid composition includes an additional inorganic salt not comprising calcium, more preferably potassium nitrate.

Thus, in a more preferred embodiment the liquid composition of the method of the invention comprises calcium nitrate and potassium nitrate; preferably the liquid composition of the invention comprises between 0.005% and 0.1% (w/v) of calcium nitrate; and/or between 0.005% and 0.05% (w/v) of potassium nitrate, where the % in (w/v) are expressed in weight with respect to the volume of the liquid composition.

In another preferred embodiment of the method of the invention, the liquid composition additionally comprises a sulphate, preferably between 0.005% and 0.05% (w/v) of a sulphate with respect to the total volume of the liquid composition, more preferably where the sulphate is magnesium sulphate.

Moreover, the liquid composition of the method of the invention may comprise other components which aid to the improvement of the pollination process.

Thus, in another preferred embodiment of the method of the invention, the liquid composition further comprises an amino acid, a polyamine, a brassinosteroid, titanium oxide, *Ascophyllum* extract, a microorganism and/or a microorganism extract.

In another preferred embodiment of the method of the invention, the liquid composition comprises an amino acid selected from the list consisting of lysine, methionine, glutamic acid, and any combination thereof. In the present invention the amino acids are, preferably, L-amino acids.

In a more preferred embodiment of the method of the invention, the liquid composition comprises between 0.5 and 6 ppm of amino acids. In the present invention the term ppm makes reference to the amount in parts per million of a compound relative to the total amount of the liquid composition.

In the present invention, the term "polyamine" refers to an organic compound which comprises at least two amino groups in its structure.

In a preferred embodiment of the method of the invention, the liquid composition comprises between 0.1% and 3% (w/v) of a polyamine, preferably where the polyamine is putrescine (NH₂(CH₂)₄NH₂).

The term "brassinosteroid" refers to a phytohormone or plant hormone belonging to the steroid group.

In a preferred embodiment of the method of the invention, the liquid composition comprises between 0.05 and 0.5 ppm of a brassinosteroid relative to the liquid composition, preferably where the brassinosteroid in brassinolide.

In a preferred embodiment of the method of the invention, the liquid composition comprises between 0.005 and 0.5 ppm of titanium oxide.

In another preferred embodiment, the liquid composition of the method of the invention comprises between 0.005 and 2.5% (w/v) of *Ascophyllum* extract relative to the total volume of the liquid composition.

In preferred embodiments, the *Ascophyllum* extract is from *Ascophyllum nodosum.*

In another preferred embodiment of the method of the invention, the liquid composition comprises a microorganism selected among a microorganism belonging to *Acinetobacter* and/or *Rosenbergiella,* preferably the composition comprises between 10³ and 10⁶ CFU/ml of the microorganism.

In another preferred embodiment of the method of the invention the microorganism extract is from a microorganism belonging to *Acinetobacter* and/or *Rosenbergiella;* preferably where the liquid composition comprises between 0.0005% and 2.5% (w/v) of an *Acinetobacter* and/or *Rosenbergiella* extract relative to the total volume of the liquid composition.

In a further preferred embodiment of the method of the invention, the liquid composition is characterized in that:
- the amino acid is selected from the group consisting of lysine, methionine, glutamic acid, or any combination thereof;
- the brassinosteroid is brassinolide;
- the polyamine is putrescine;
- an *Ascophyllum* extract;
- the microorganism belongs to *Acinetobacter or Rosenbergiella*; and/or
- the extract is from a microorganism which belongs to *Acinetobacter* or *Rosenbergiella.*

In preferred embodiments, the microorganisms and microorganism extracts from *Acinetobacter* and/or *Rosenbergiella,* are, respectively, from *Acinetobacter boissieri* and *Rosenbergiella nectarea.*

In another preferred embodiment of the method of the invention, the liquid composition comprises between 1 and 5 mg of pollen per millilitre of liquid.

In another preferred embodiment of the method of the invention, the liquid composition is an aqueous composition. Thus, the different elements of the composition are comprised in water or an aqueous solution.

The term "plant" refers to any living organism of the kingdom Plantae, preferably any living organism belonging to any genus/species of the kingdom Plantae. In the present invention the term plant includes both an individual plant and a set of plants, for example crop plants.

In a preferred embodiment of the method of the invention, the plant is at the flowering stage. Preferably, the mixture of step a) is applied on the flowers of the plant.

In the present invention, the term "apply" or "administer", used interchangeably, refer to the method or procedure by which a plant is brought into contact with the liquid composition of the method of the invention.

In the present invention the application of the liquid composition is specifically performed by means of electrospraying. Electrospraying is a process which combines liquid atomization (like pesticides, fertilizers, or paint) with the application of an electric voltage in order to confer electric charge to the pulverization particles. As the expert in the subject matter knows, particle size conditions, application distance, and applied voltage can be defined routinely.

In another preferred embodiment of the method of the invention, in the electrospraying of step (b) a voltage of between 10,000 and 15,000 volts is applied, preferably at low amperage.

In another preferred embodiment of the method of the invention, the electrospraying particle size ranges between 80 and 120 microns, more preferably 100 microns.

In another preferred embodiment of the method of the invention, the application of the liquid composition by means of electrospraying is performed at a distance ranging between 0.5 and 5 meters. Preferably the application is performed on the flowers of the plant.

In another preferred embodiment of the method of the invention, the electrospraying is performed by applying the liquid composition in an amount ranging between 40 and 80 L/ha (liters of liquid composition per surface hectare of a surface with plants).

In another preferred embodiment of the method of the invention, the electrospraying is performed by applying an amount ranging between 0.5-2 g/ha (pollen grams per surface hectare of a surface with plants).

The electrospraying can be performed using an eletropulveriser (electrostatic nebuliser). Suitable sprayers for agricultural applications are available on the market, for instance the Martignani sprayer of the model WHIRLWIND SHUTTLE M612 - Turbo2.

In another preferred embodiment of the method of the invention, the plant is selected from the list which consists of almond, avocado, pistachio, olive, passion fruit, cherry, plum, sunflower, and date palm.

In another preferred embodiment of the method of the invention, the liquid composition, from now on "liquid composition A", comprises:
- between 12 and 28% (w/v) of sucrose,
- between 0.005 and a 0.10% (w/v) of calcium nitrate,
- between 0.003 and 0.10% (w/v) of boric acid,
- between 0.005 and 0.05% (w/v) of potassium nitrate,
- between 0.005 and 0.05% (w/v) of magnesium sulphate,
- between 0.005 and 2.5% (w/v) of *Ascophyllum nodosum* extract,
- between 0.005 and 0.5 ppm of titanium oxide,
- between 0.1 and 3% (w/v) of putrescine,
- between 10³and 10⁶ CFU/ml of *Rosenbergiella nectarea,*
- between 0.5 and 6 ppm of lysine, and
- between 0.5 and 6 ppm of methionine.

In another preferred embodiment of the method of the invention, the liquid composition A is applied to an almond plant or to a group of almond plants.

In another preferred embodiment of the method of the invention, the liquid composition, from now on "liquid composition B", comprises:
- between 12 and 28% (w/v) of sucrose,
- between 0.005 and a 0.10% (w/v) of calcium nitrate,
- between 0.003 and 0.10% (w/v) of boric acid,
- between 0.005 and 0.05% (w/v) of potassium nitrate,
- between 0.005 and 0.05% (w/v) of magnesium sulphate,
- between 0.005 and 2.5% (w/v) of *Ascophyllum nodosum* extract,
- between 0.005 and 0.5 ppm of titanium oxide,
- between 0.1 and 3% (w/v) of putrescine,
- between 0.05 and 0.5 pm of brassinolide,
- between 0.5 and 6 ppm of lysine, and
- between 0.5 and 6 ppm of methionine.

In another preferred embodiment of the method of the invention, the liquid composition B is applied to an avocado plant or to a group of avocado plants.

In another preferred embodiment of the method of the invention, the liquid composition, from now on "liquid composition C", comprises:
- between 12 and 28% (w/v) of sucrose,
- between 0.005 and a 0.10% (w/v) of calcium nitrate,
- between 0.003 and 0.10% (w/v) of boric acid,
- between 0.005 and 0.05% (w/v) of potassium nitrate,
- between 0.005 and 0.05% (w/v) of magnesium sulphate,
- between 0.005 and 0.5 ppm of titanium oxide,
- between 0.1 and 3% (w/v) of putrescine,
- between 0.5 and 6 ppm of lysine,
- between 0.5 and 6 ppm of methionine, and
- between 0.5 and 6 ppm of glutamic acid.

In another preferred embodiment of the method of the invention, liquid composition C is applied to a pistachio plant or to a group of pistachio plants.

In another preferred embodiment of the method of the invention, the liquid composition, from now on "liquid composition D", comprises:
- between 12 and 28% (w/v) of sucrose,
- between 0.005 and 0.10% (w/v) of calcium nitrate,
- between 0.003 and 0.10% (w/v) of boric acid,
- between 0.005 and 0.05% (w/v) of potassium nitrate,
- between 0.005 and 0.05% (w/v) of magnesium sulphate,
- between 0.05 and 0.5 ppm of brassinolide, and
- between 0.5 and 6 ppm of methionine.

In another preferred embodiment of the method of the invention, liquid composition D is applied to a date palm plant or to a group of date palm plants.

In another preferred embodiment of the method of the invention, the liquid composition, from now on "liquid composition E", comprises :
- between 12 and 28% (w/v) of sucrose,
- between 0.005 and 0.10% (w/v) of calcium nitrate,
- between 0.003 and 0.10% (w/v) of boric acid,
- between 0.005 and 2.5% (w/v) of *Ascophyllum nodosum* extract,
- between 0.005 and 0.5 ppm of titanium oxide,
- between 0.1 and 3% (w/v) of putrescine,
- between 0.5 and 6 ppm of lysine, and
- between 0.5 and 6 ppm of methionine.

In another preferred embodiment of the method of the invention, liquid composition E is applied to olive plants or to a group of olive plants.

In another preferred embodiment of the method of the invention, the liquid composition, from now on "liquid composition F", comprises:
- between 12 and 28% (w/v) of sucrose,
- between 0.005 and 0.10% (w/v) of calcium nitrate,
- between 0.003 and 0.10% (w/v) of boric acid,
- between 0.005 and 0.05% (w/v) of potassium nitrate, and
- between 0.005 and 0.05% (w/v) of magnesium sulphate.

In another preferred embodiment of the method of the invention, liquid composition F is applied to a passion fruit plant or to a group of passion fruit plants.

In another preferred embodiment of the method of the invention, the liquid composition, from now on "liquid composition G", comprises:
- between 12 and 28% (w/v) of sucrose,
- between 0.005 and 0.10% (w/v) of calcium nitrate,
- between 0.003 and 0.10% (w/v) of boric acid,
- between 0.005 and 0.05% (w/v) of potassium nitrate,
- between 0.005 and 0.05% (w/v) of magnesium sulphate,
- between 0.005 and 0.5 ppm of titanium oxide,
- between 0.1 and 3% (w/v) of putrescine,
- between 0.05 and 0.5 ppm of brassinolide,
- between 0.5 and 6 ppm of lysine, and
- between 0.5 and 6 ppm of methionine.

In another preferred embodiment of the method of the invention, liquid composition G is applied to a cherry plant or to a group of cherry plants.

In another preferred embodiment of the method of the invention, the liquid composition, from now on "liquid composition H", comprises:
- between 12 and 28% (w/v) of sucrose,
- between 0.005 and 0.10% (w/v) of calcium nitrate,
- between 0.003 and 0.10% (w/v) of boric acid,
- between 0.005 and 0.05% (w/v) of potassium nitrate,
- between 0.005 and 0.05% (w/v) of magnesium sulphate,
- between 0.005 and 0.5 ppm of titanium oxide,
- between 0.1 and 3% (w/v) of putrescine, and
- between 0.05 and 0.5 ppm of brassinolide.

In another preferred embodiment of the method of the invention, liquid composition H is applied to a plum plant or to a group of plum plants.

In another preferred embodiment of the method of the invention, the liquid composition, from now on "liquid composition I", comprises:
- between 12 and 28% (w/v) of sucrose,
- between 0.005 and 0.10% (w/v) of calcium nitrate,
- between 0.003 and 0.10% (w/v) of boric acid,
- between 0.005 and 0.05% (w/v) of potassium nitrate,
- between 0.005 and 0.05% (w/v) of magnesium sulphate,
- between 0.005 and 0.5 ppm of titanium oxide, and
- between 0.1 and 3% (w/v) of putrescine.

In another preferred embodiment of the method of the invention, liquid composition I is applied to a sunflower plant or to a group of sunflower plants.

In all preferred embodiments referring to the liquid composition, the quantities of the different components are expressed relative to the total of the liquid composition.

In another aspect, the invention relates to a liquid composition for pollination, hereinafter referred to as the "composition of the invention", where said composition comprises an osmotic component, a calcium source, a boron source, and at least one component selected from the list consisting of an amino acid, a polyamine, a brassinosteroid, titanium oxide, an *Ascophyllum* extract, a microorganism from the *Acinetobacter* or *Rosenbergiella,* and/or an extract derived from *Acinetobacter* or *Rosenbergiella.*

In another preferred embodiment, the composition of the invention comprises between 12 and 28% (w/v) of an osmotic component; between 0.005 and 0.10% (w/v) of a calcium source; and/or between 0.003-0.10% (w/v) of a boron source.

In another preferred embodiment of the composition of the invention, the osmotic component of the liquid composition is a carbohydrate, the calcium source is an inorganic salt, and/or the boron source is boric acid.

In another preferred embodiment, the composition of the invention comprises between 12 and 28% (w/v) of a carbohydrate; between 0.005 and 0.10% (w/v) of calcium inorganic salt; and/or between a 0.003 and 0.10% (w/v) of boric acid, where the % in (w/v) are expressed in weight relative to the volume of the liquid composition.

In another preferred embodiment of the composition of the invention, the osmotic component of the liquid composition is a carbohydrate selected from a monosaccharide, a disaccharide, or a combination thereof; the carbohydrate of the liquid composition is selected from a list consisting of sucrose, fructose, glucose, or a combination thereof, preferably where the carbohydrate is sucrose.

In another preferred embodiment of the composition of the invention, the inorganic calcium salt is calcium nitrate.

In another preferred embodiment, the composition of the invention comprises another inorganic salt, preferably an inorganic salt not comprising calcium, more preferably potassium nitrate.

In another preferred embodiment, the composition of the invention comprises calcium nitrate and potassium nitrate; preferably the liquid composition of the invention comprises between 0.005 and 0.1 % (w/v) of calcium nitrate and/or between a 0.005 and 0.05 % (w/v) of potassium nitrate, where % in (w/v) are expressed in weight relative to the volume of the liquid composition.

In another preferred embodiment, the composition of the invention comprises a sulphate, preferably comprises between 0.005 and 0.05 % (w/v) of a sulphate relative to the total volume of the liquid composition, more preferably where the sulphate is magnesium sulphate.

In another preferred embodiment of the composition of the invention the amino acid is selected from a list consisting of lysine, methionine, glutamic acid, or any combinations thereof.

In another preferred embodiment, the composition of the invention comprises between 0.5 and 6 ppm of amino acids.

In another preferred embodiment, the composition of the invention comprises between 0.1 and a 2 % (w/v) of a polyamine, preferably where the polyamine is putrescine (NH₂(CH₂)₄NH₂).

In another preferred embodiment, the composition of the invention comprises between 0.05 and 0.5 ppm of a brassinosteroid relative to the liquid composition, preferably where the brassinosteroid is brassinolide.

In another preferred embodiment, the composition of the invention comprises between 0.005 and 0.5 ppm of Titanium oxide.

In another preferred embodiment, the composition of the invention comprises between 0.005 and 2.5 % (w/v) of *Ascophyllum* extract relative to the total volume of the liquid composition.

In another preferred embodiment, the composition of the invention comprises a microorganism selected from a microorganism belonging to between *Acinetobacter* and/or *Rosenbergiella* at an amount ranging between 10³and 10⁶ CFU/ml of the microorganism.

In another preferred embodiment, the composition of the invention comprises between 0.0005 and 2.5 % (w/v) of an *Acinetobacter* and/or *Rosenbergiella* extract relative to the total volume of the liquid composition.

In another preferred embodiment, the composition of the invention is characterized by:
- the amino acid is selected from the list consistin of lysine, methionine, glutamic acid, and any combinations thereof;
- the brassinosteroid is brassinolide;
- the polyamine is putrescine;
- an *Ascophyllum* extract;
- the microorganism belongs to *Acinetobacter or Rosenbergiella*; and/or
- the extract is from a microorganism which belongs to *Acinetobacter* or *Rosenbergiella.*

In another preferred embodiment, the composition of the invention comprises the components according to any of the compositions A, B, C, D, E, F, G, H or I from the method of the invention.

In another preferred embodiment, the composition of the invention is characterised by comprising pollen.

In another preferred embodiment, the composition of the invention comprises between 1 and 5 mg of pollen/ millilitre of liquid composition.

In another preferred embodiment of the composition of the invention, the liquid composition is an aqueous composition. Thus, the different elements of the composition are preferably comprised in water or an aqueous solution.

In another aspect, the invention relates to the use of the composition of the invention in the pollination of a plant; preferably where the plant is selected from the list consisting of almond, avocado, pistachio, olive, passion fruit, cherry, plum, sunflower, and date palm.

### DESCRIPTION OF THE FIGURES

**Fig. 1****. Avocado plant yield following pollination.**
**Fig. 2****. Fruit yield per avocado plant.**

### EXAMPLES

Below, the invention is illustrated by a series of tests carried out by the inventors, which demonstrate the improvement in crop yield resulting from pollination using the method of the invention.

### Example 1. Avocado Pollination

Trial Data. The trial was conducted on a farm located in Vélez-Málaga (Málaga, Spain). The farm comprises 10 hectares of the Hass cultivar, with 15% of the trees corresponding to the pollinator cultivar Fuerte. Year of planting: 2015.

Materials and Methods. Six randomized blocks of 1.5 hectares each were established. Three blocks were left for natural pollination, and three were artificially pollinated using the method of the invention. Male-phase flowers from the Fuerte cultivar were collected manually until a volume of 0.5 L was obtained for pollinating 4.5 hectares. Pollen was extracted using a biphasic liquid extraction method. First, 400 mL of cyclohexane was added and stirred for 45 seconds. The mixture was filtered through a 0.2 mm mesh. After filtration, 200 mL of aqueous solution (20% sucrose, 0.2 g/L calcium nitrate, 0.2 g/L boric acid) was added. Both phases were mixed vigorously for 1 minute and allowed to settle for 5 minutes. The bottom phase, containing the isolated pollen, was recovered.

Pollination was performed in a single pass when the field reached 60% bloom. The pollination matrix was prepared for 4.5 hectares, divided into three 1.5-hectare blocks. A Martignani WHIRLWIND SHUTTLE M612 - Turbo2 -1500 L with tower sprayer was used. The tank was filled with 5 L of avocado matrix and 310 L of water to produce the final matrix (15% sucrose, 0.01% boric acid, 0.03% potassium nitrate, 0.01% calcium nitrate, 0.02% magnesium sulfate, 0.001% *Ascophyllum* extract, 0.2 ppm titanium oxide, 2 ppm lysine, and 0.3 ppm brassinolide). A total of 200 mL of the extracted pollen was added, mixed thoroughly, and applied at 70 L/ha, 5.5 km/h, using electrostatic spraying.

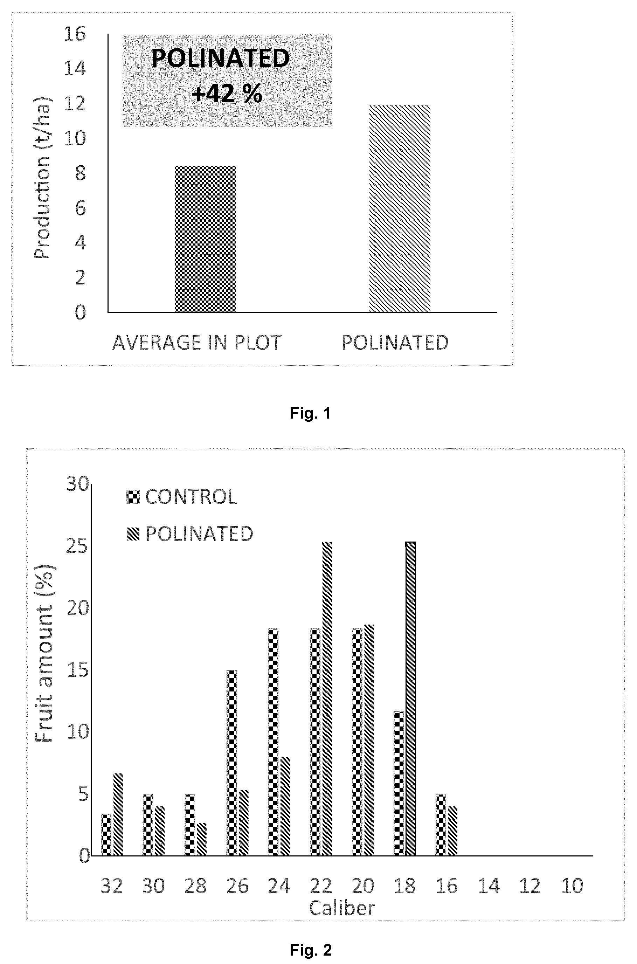

Results. At harvest, yield and fruit quality were evaluated. The average yield in the naturally pollinated blocks was 8.41 T/ha, while the blocks pollinated with the invention method yielded 11.92 T/ha (a 42% increase in production, figure 1).

Fruit size (caliber) analysis revealed no significant differences between naturally and artificially pollinated fruits, indicating that the 42% increase in yield did not compromise fruit size (see figure 2).

### Example 2. Almond Pollination

Trial Data. The trial was conducted on a farm located in Castelo-Branco (Portugal). The farm consists of 120 hectares of Guara, Soleta, Penta, and Avijor cultivars. Year of planting: 2019.

Materials and Methods. Six randomized 3-hectare blocks were established for the Penta cultivar. Three blocks were left for natural pollination, and three were artificially pollinated using the method of the invention. The same design was applied for the Guara cultivar. Flowers from Soleta and Avijor were collected using a mechanical shaker bar, yielding 20 L of floral material. Flowers were gradually dried in a hot-air oven (12 h at 22°C, followed by 6 h at 25°C). Dried flowers were placed in a rotary sieve with 0.02 mm mesh for 2 hours to extract pollen, which was then stored in 0.2 g cryovials.

The pollination matrix was prepared for 18 hectares divided in six 3-hectare blocks (two cultivars, Penta and Guara). A Martignani WHIRLWIND SHUTTLE M612 - Turbo2 -1500 L sprayer was used. The tank was filled with 18 L of almond matrix and 882 L of water to obtain the final matrix (25% sucrose, 0.03% boric acid, 0.02% potassium nitrate, 0.02% calcium nitrate, 0.02% magnesium sulfate, 0.05% *Ascophyllum* extract, 0.5 ppm titanium oxide, 0.1 ppm putrescine, and 10⁴ CFU/mL *Rosenbergiella*)*.* Pollen was added at 0.2 grams per hectare, and the mixture was applied at 50 L/ha, 6.5 km/h, using electrostatic spraying.

Results. At harvest, yields were measured. For the Penta cultivar, the naturally pollinated blocks yielded an average of 1097 kg/ha, while the blocks using the invention method yielded 1325 kg/ha, corresponding to a 20.78% increase. For the Guara cultivar, natural pollination yielded 1851 kg/ha, while assisted pollination yielded 2107 kg/ha, a 13.83% increase.

### Example 3. Pistachio Pollination

Trial Data. The trial was conducted on a farm in Antequera (Málaga, Spain) with 60 hectares planted with Kerman (female) and Peter (male) cultivars.

Materials and Methods. Six randomized 1-hectare blocks were established for the *Kerman* cultivar. Three blocks were left for natural pollination, and three were artificially pollinated using the method of the invention. Pollen from the Peter cultivar was collected using aspiration and density purification, then stored in 0.2 g cryovials.

The pollination matrix was prepared for 6 hectares (six 1-hectare blocks). A Martignani WHIRLWIND SHUTTLE M612 - Turbo2 -1500 L sprayer was used. The tank was filled with 6 L of pistachio matrix and 294 L of water, and the solution was applied at 50 L/ha, 6.5 km/h, using electrostatic spraying.

Results. At harvest, yield was assessed. The naturally pollinated Kerman blocks yielded an average of 698 kg/ha, while the blocks using the invention method yielded 725 kg/ha, along with a 66% reduction in the proportion of empty fruits.

## Claims

1. A method for pollinating a plant, comprising the following steps:
a) Mixing pollen with a liquid composition; and
b) Applying the mixture from step a) to the plant via electrospraying; wherein the liquid composition comprises an osmotic component, a calcium source, and a boron source.

2. The method according to claim 1, wherein the liquid composition comprises between 12% and 28% (w/v) of an osmotic component; between 0.005% and 0.10% (w/v) of the calcium source; and/or between 0.003% and 0.10% (w/v) of the boron source.

3. The method according to claim 1 or 2, wherein the osmotic component of the liquid composition is a carbohydrate, the calcium source is an inorganic calcium salt, and/or where the boron source is boric acid.

4. The method according to any of claims 1 to 3, wherein the osmotic component is a carbohydrate selected from a monosaccharide, a disaccharide, or a combination thereof; preferably, the carbohydrate is selected from the list consisting of sucrose, fructose, glucose, or a combination thereof, more preferably sucrose.

5. The method according to any of claims 1 to 4, wherein the calcium source is calcium nitrate.

6. The method according to any of claims 1 to 5, wherein the composition comprises an additional inorganic salt, preferably potassium nitrate.

7. The method according to any of claims 1 to 5, wherein the liquid composition further comprises a sulphate, preferably magnesium sulphate.

8. The method according to any of claims 1 to 7, wherein the liquid composition further comprises an amino acid, a polyamine, a brassinosteroid, titanium oxide, an *Ascophyllum* extract, a microorganism, and/or an extract of a microorganism.

9. The method according to claim 8, wherein:
- the amino acid is selected from the list consisting of lysine, methionine, glutamic acid, or any combination thereof;
- the brassinosteroid is brassinolide;
- the polyamine is putrescine;
- an *Ascophyllum* extract;
- the microorganism belongs to *Acinetobacter or Rosenbergiella*; and/or
- the extract is from a microorganism from *Acinetobacter* or *Rosenbergiella.*

10. The method according to any of claims 1 to 9, wherein the liquid composition comprises between 1 and 5 mg of pollen/ millilitre of liquid composition.

11. The method according to any of claims 1 to 10, wherein the plant is a cultivated plant, preferably the plant is selected from the list consisting of almond, avocado, pistachio, olive, passion fruit, cherry, plum, sunflower, and date palm.

12. A liquid composition for pollination comprising an osmotic component, a calcium source, a boron source, and at least one component selected from the group consisting of an amino acid, a polyamine, a brassinosteroid, titanium oxide, an *Ascophyllum* extract, a microorganism from *Acinetobacter or Rosenbergiella,* and/or an extract of a microorganism from *Acinetobacter* or *Rosenbergiella.*

13. The liquid composition according to claim 11 **characterized in that** it comprises pollen.

14. Use of the liquid composition according to claims 11 or 12 in the pollination of a plant.

15. The use according to claim 14, wherein the plant is selected from the list consisting of almond, avocado, pistachio, olive, passion fruit, cherry, plum, sunflower, and date palm.
